Europäisches Patentamt

European Patent Office

Office européen des brevets

(19)

(11) Veröffentlichungsnummer: **0 004 039**
**B1**

(12) **EUROPÄISCHE PATENTSCHRIFT**

(45) Veröffentlichungstag der Patentschrift: **17.06.81**

(21) Anmeldenummer: **79100613.3**

(22) Anmeldetag: **02.03.79**

(51) Int. Cl.³: **B 05 B 17/06,**
**A 61 M 15/00**

(54) **Flüssigkeitszerstäuber.**

(30) Priorität: **15.03.78 DE 2811248**

(43) Veröffentlichungstag der Anmeldung:
**19.09.79 Patentblatt 79/19**

(45) Bekanntmachung des Hinweises auf die Patenterteilung:
**17.06.81 Patentblatt 81/24**

(84) Benannte Vertragsstaaten:
**CH FR GB IT NL SE**

(56) Entgegenhaltungen:
**DE - A - 2 129 665**
**DE - A - 2 656 370**
**FR - A - 2 196 592**
**FR - A - 2 321 907**
**US - A - 3 103 310**

(73) Patentinhaber: **Bosch-Siemens Hausgeräte GmbH**
**Stuttgart**
**Patent- und Vertragswesen Hochstrasse 17**
**D-8000 München 80 (DE)**

(72) Erfinder: **Eck, Walter, Dipl. Phys.**
**Waldgartenstrasse 65**
**D-8000 München 70 (DE)**

Courier Press, Leamington Spa, England.

Flüssigkeitszerstäuber

Die Erfindung geht aus von einem Flüssigkeitszerstäuber gemäß dem Oberbegriff des Patentanspruches 1.

Bei einem bekannten Flüssigkeitszerstäuber (US—PS 3 103 310) ist für den Transport der zu zerstäubenden Flüssigkeit aus einem Flüssigkeitsbehälter heraus eine Flüssigkeitszuführung vorgesehen, bestehend aus einem drehbaren oder förderbandartig bewegbaren Verteilerelement und aus einem Flüssigkeits-Zuführdocht, wobei der Docht seine Flüssigkeit außerhalb des Zerstäuberbereiches an das Verteilerelement abgibt, wonach das Verteilerelement die zugeführte Flüssigkeit bis in den Bereich eines Schwingungsgenerators bringt, von welchem aus die Flüssigkeit zerstäubt wird. Von Nachteil hierbei ist der Umstand, daß man für die Bewegung des Verteilerelementes Antriebs- und Lagervorrichtungen benötigt und daß sich bei längerer Betriebsdauer der am Verteilerelement schleifend anliegende Docht allmählich abnützt, wodurch der exakte Flüssigkeitsübergang gefährdet ist. Nicht zuletzt kann sich bei diesem bekannten Flüssigkeitszerstäuber auch die Schwierigkeit ergeben, daß die vom Docht übernommene Flüssigkeit sich während der Zuführbewegung auf dem Verteilerelement zu größeren Tropfen sammelt, wodurch eine sehr feine nebelartige Zerstäubung in Frage gestellt ist.

Bei einem anderen bekannten Flüssigkeitszerstäuber (FR—A—2321907) ist ein Flüssigkeitsbehälter oberhalb einer Zerstäuberplatte angeordnet. Dieser Flüssigkeitsbehälter besitzt einen unteren Auslaufkanal, vor dem ein netzartiges Gebilde angeordnet ist, das die Zerstäberplatte vollständig überzieht und als Flüssigkeitszuführung unter Ausnützung der Kapillarwirkung des netzartigen Gebildes dient. Hierbei besteht die Schwierigkeit, daß des netzartige Gebilde eine relativ große Dämpfungsmasse darstellt die imstande ist, derart dämpfend auf die Zerstäuberplatte einzuwirken, daß eine Flüssigkeitszerstäubung nicht mehr stattfindet.

Der Erfindung liegt die Aufgabe zugrunde, einen Flüssigkeitszerstäuber der eingangs genannten Art so auszugestalten, daß die Flüssigkeitszuführung vereinfacht und die Zerstäubung verbessert wird.

Diese Aufgabe wird gelöst durch die im Kennzeichnungsteil des Patentanspruches 1 aufgeführten Merkmale.

Im Gegensatz zu dem erstgenannten bekannten Flüssigkeitszerstäuber wird hierbei für die Zuführung und für die Bereitstellung der Flüssigkeit nur ein einziges Element, nämlich der Docht benötigt, was nicht nur eine konstruktive Vereinfachung darstellt, sondern was auch sicherstellt, daß sämtliche aus dem Flüssigkeitsbehälter entnommene Flüssigkeit auch tatsächlich zum Zerstäuberelement gelangt, wobei die Bildung größerer Tropfen am Dochtende vermieden wird. Hierbei ist im Vergleich zum zweitgenannten bekannten Flüssigkeitszerstäuber eine, die Schwingbewegung unterbrechende Dämpfung des Zerstäuberelementes nicht zu befürchten. Vielmehr schwingt das freie Dochtende mit mehr oder weniger gedämpfter Schwingungsamplitude mit dem Zerstäuberelement mit, wobei sich insgesamt eine besonders feine, nebelartige Zerstäubung der Flüssigkeit ergibt.

Eine vorbestimmte Lage und eine sichere mechanische Ankopplung des Dochtendes am Zerstäuberelement läßt sich durch die Lösung des Patentanspruches 2 sicher erreichen.

Gemäß einer weiteren Ausgestaltung der Erfindung weist der vorzugsweise durchsichtige Flüssigkeitsbehälter eine als Einfullöffnung sowie als Einführöffnung für den Docht bzw. sein Dochtrohr dienende Öffnung auf und ist zusammen mit dem Docht aus dem Gehäuse des Flüssigkeitszerstäubers herausnehmbar und in dieses einsetzbar. Vorzugsweise ist hierbei die Öffnung im Flüssigkeitsbehälter so eng, daß zwischen Öffnungsrand und Docht bzw. Dochtrohr auch bei Schräglage des Gerätes keine Flüssigkeit unbeabsichtigt ausfließen kann. Der Flüssigkeitszerstäuber ist damit völlig lageunabhängig, kann z.B. als Inhalationsgerät auch im Liegen benützt werden. Insbesondere besteht im Rahmen der Erfindung auch die Möglichkeit, komplette, handelsfähige Baueinheiten, bestehend aus Flüssigkeitsbehälter und eingesetztem Docht und Zerstäuberflüssigkeit bereitzustellen, die in sehr einfacher Weise in den Flüssigkeitszerstäuber, z.B. in das Inhalationsgerät, in das Raumspray-Gerät und dergleichen eingesetzt werden können.

Eine gute, gleichbleibend exakte Anlage des Dochtes am Zerstäuberelement ist gemäß einer anderen Ausgestaltung der Erfindung dadurch gewährleistet, daß ein auf das Gehäuse des Flüssigkeitszerstäubers aufsetzbarer, vorzugsweise trichterartiger Sprühkopf für das Zerstäuberelement und für den am Zerstäuberelement anliegenden Docht eine Aussparung aufweist mit einer Begrenzungskante, welche den Docht oder sein Dochtrohr gegen das Zerstäuberelemer⁺ drückt.

Die Erfindung wird anhand eines Inhalationsgerätes zur Behandlung der Atemwege verdeutlicht. Beim Ausführungsbeispiel ist auf die schräg geneigte Frontseite 1 eines Gerätegehäuses 2 ein mit 3 bezeichneter, als Inhalationstrichter ausgeführter Sprühkopf aufsetzbar, z.B. in Pfeilrichtung aufsteckbar. Im Innenraum 4 des Gerätegehäuses 2 sind an einer steckbaren Schaltungsplatte 5 durch eine Anschlußöffnung 6 hindurch zugängliche elektrische Anschlußelemente 7 mit Anschlußsteckern 8 sowie ein allgemein mit 9 bezeichneter Schwingungsgenerator für Ultraschall-Biegeschwingungen befestigt. Der nicht weiter erläuterte, z.B. mit einer Niederspannunganregungselektronik ausgestattete Schwingungsgenerator 9 ist über Leitungen 10 an einen kegelförmigen Schallübertrager 11 angeschlossen, welcher eine piezokeramische Schicht 12 sowie an dem, dem Sprühkopf 3 zugewandten Ende eine Zerstäuberplatte 13 aufweist. Die an der Schicht 12 erfolgten Ultraschall-Biegeschwingungen werden auf die Zerstäuberplatte 13 übertragen. Am unteren Ende der Gerätefrontseite 1 ist ein Hohlraum 15 vorgesehen, innerhalb welchem eine wannenartige Aufnahme 16 für einen Flüssigkeitsbehälter 17 angeordnet ist. Der Hohlraum 15 wird durch Aufstecken des Sprühkopfes 3 nach außen hin abgedeckt. In die wannenartige Aufnahme 16 einsetzbar ist ein z.B. aus durchsichtigem Kunststoffmaterial bestehender Flüssigkeitsbehälter 17, der am oberen Ende eine Flanschöffnung 18 besitzt, durch welche hindurch ein zum größtem Teil von einem Dochtrohr 19 umgebener Docht 20 in das Innere des Flüssigkeitsbehälters 17 eingeführt ist. Der Docht reicht einerseits bis auf den Grund des Flüssigkeitsbehälters 17 und liegt mit seinem anderen, uber das Dochtrohr 19 hinausragenden freien Dochtende mit Vorspannung an einer Begrenzungskante des plattenartigen Zerstäuberelementes 13 an. Das weitgehend formstabile Dochtrohr 19 hält den asu zumindest weitgehend elastischem Material z.B. aus einem textilen Gewebe bestehenden Docht 20 in einer vorbestimmten Stellung. Der als Inhalationstrichter ausgebildete Sprühkopf 3 besitzt in seiner Grundplatte 21 eine runde Öffnung 22 für die Zerstäuberplatte 13 sowie eine in die letztgenannte Öffnung einmündende längliche Öffnung 23 für das freie Ende des Dochtes 20 bzw. dessen Dochtrohr 19. Die letztgenannte, längliche Öffnung 23 besitzt eine schräge Begrenzungskante 24, die mit dem Aufsetzen des Sprühkopfes 3 auf das Gerätegehäuse 2 gegen das Dochtrohr 19 drückt und damit das freie Dochtende 20 elastisch gegenüber der Zerstäuberplatte 13 verspannt, sodaß eine gleichbleibend exakte mechanische Ankopplung zwischen Zerstäuberplatte und Docht gewährleistet ist. Flüssigkeitsbehälter 17, Dochtrohr 19 und Docht 20 können als fertige, handelsfähige Baueinheit auf Lager gehalten und bei Bedarf in das Gerät eingesetzt werden. Sobald der Docht 20 mit der Flüssigkeit 25 in Berührung kommt, wird diese Flüssigkeit entlang des Dochtes infolge Kapillarwirkung zum freien Dochtende hin transportiert und wird infolge der hochfrequenten Schwingungen der Zerstäuberplatte 13 und somit auch des Dochtendes zum Sprühkopf 3 hin in sehr feiner Verteilung von beispielsweise wenigen tauselstel Millimeter pro Sprühteilchen zerstäubt. Der Flüssigkeitstransport erfolgt ohne weiteres Zutun, insbesondere ohne zusätzlichen Energieaufwand ununterbrochen bis zur völligen Entleerung des Flüssigkeitsbehälters 17. Hierbei erfolgt dieser Flüssigkeitstransport entsprechend dem Bedarf, d.h. entsprechend der Zerstäubung der fein zerteilten Flüssigkeit.

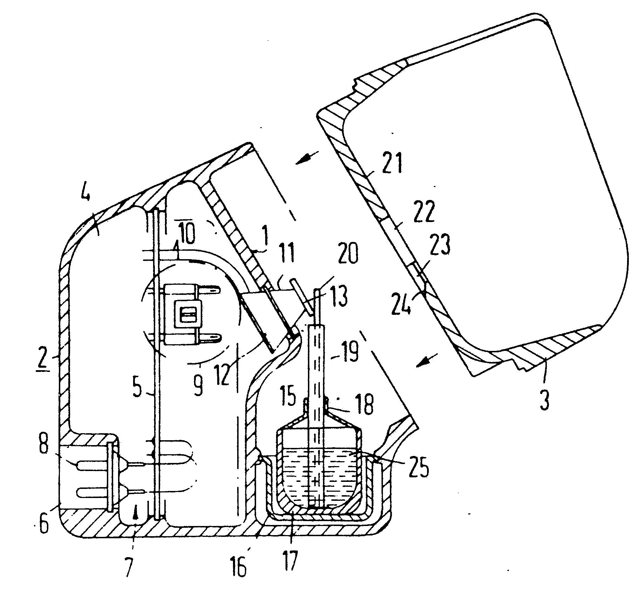

Selbstverständlich ist jede andere Anordnung und Ausgestaltung des Flüssigkeitsbehälters im Rahmen der Erfindung denkbar. So besteht die Möglichkeit, den transparenten Flüssigkeitsbehälter in einer nischenartigen Ausnehmung des Gerätegehäuses anzuordnen, derart, daß zum einen das Einsetzen des Behälters noch mehr erleichtert wird und jederzeit der Flüssigkeitsstand ersichtlich ist. Dies ist insbesondere dann von Vorteil, wenn der Flüssigkeitszerstäuber als Raumspraygerät oder dergleichen ausgebildet ist, wobei dann vorzugsweise größere, für eine Vielzahl von Sprühvorgängen ausreichende Flüssigkeitsbehälter verwendet werden. In der Zeichnung ist der Übersichtlichkeit halber weiter dargestellt, daß an der Peripherie des Gerätegehäuses 2 ein z.B. als Druckschalter ausgebildeter Schalter vorgesehen ist, durch welchen das Gerät in Betrieb genommen bzw. außer Betrieb gesetzt werden kann.

## Patentansprüche

1. Flüssigkeitszerstäuber mit einem Schwingungsgenerator vorzugsweise einen Ultraschall-Biegeschwingungen erzeugenden Schwingungsgenerator (9) und einer mit diesem gekoppelten Zerstäuberplatte (13), mit einem Flüssigkeitsbehälter (17) sowie mit einem zwischen Flüssigkeitsbehälter und Zerstäuberplatte angeordneten, der Flüssigkeitszuführung durch Kapillarwirkung dienenden, flexiblen Element (20), dadurch gekennzeichnet, daß dieses Element ein aus elastisch nachgiebigem Material bestehender Docht (20) ist, der mit seinem freien Ende mit mechanischer Vorspannung an der Zerstäuberplatte (13) anliegt.

2. Flüssigkeitszerstäuber nach Anspruch 1, dadurch gekennzeichnet, daß der Docht (20) bis unmittelbar vor seiner Ankoppelstelle in einem weitgehend formstabilen Dochtrohr (19) verlegt und geführt ist.

3. Flüssigkeitszerstäuber nach Anspruch 1 oder 2, dadurch gekennzeichnet, daß der Docht (20) punkt- bzw. linienförmig an der Zerstäuberplatte (13) anliegt.

4. Flüssigkeitszerstäuber nach Anspruch 3, dadurch gekennzeichnet, daß der Docht (20) mit mechanischer Vorspannung an einer Begrenzungskante der Zerstäuberplatte (13) anliegt.

5. Flüssigkeitszerstäuber nach einem oder mehreren der vorhergehenden Ansprüch, dadurch gekennzeichnet, daß der vorzugsweise durchsichtige Flüssigkeitsbehälter (17) eine als Einfüllöffnung sowie als Einführöffnung für den Docht (20) bzw. sein Dochtrohr (19) dienende Öffnung (18) aufweist und zusammen mit dem Docht aus dem Gehäuse (2) des Flüssigkeitszerstäubers herausnehmbar und in dieses einsetzbar ist.

6. Flüssigkeitszerstäuber nach einem oder mehreren der vorhergehenden Ansprüche, dadurch gekennzeichnet, daß ein auf das Gehäuse (2) des Flüssigkeitszerstäubers aufsetzbarer vorzugsweise trichterartiger Sprühkopf (3) für die Zerstäuberplatte (13) und für den an der Zerstäuberplatte anliegenden Docht (20) eine Aussparung (22/23) aufweist mit einer Begrenzungskante (24), welche den Docht gegen die Zerstäuberplatte drückt.

**Revendications**

1. Pulvérisateur pour produits liquides à générateur d'oscillations de préférence un générateur d'oscillations (9) fournissant des oscillations ultrasonores de flexion et à plaque de pulvérisation (13) couplées audit générateur (9), du type comportant un réservoir à liquide (17) ainsi qu'un élément flexible (20) disposé entre le réservoir à liquide et la plaque de pulvérisation et servant à amener le liquide par capilarité, caractérisé par le fait que ledit élément est constitué par une mèche (20) faite avec un matériau élastiquement flexible qui porte par son extrémité libre, avec une tension mécanique préalable, contre la plaque de pulvérisation (13).

2. Pulvérisateur selon la revendication 1, caractérisé par le fait que la mèche (20) s'étend et est guidée dans un tube (12), largement stable du point de vue de sa forme, dans sa partie qui est située directement avant sa partie couplée audit générateur.

3. Pulvérisateur selon la revendication 1 ou 2, caractérisé par le fait que la mèche (20) porte contre la plaque de pulvérisation (13) suivant un point ou une droite.

4. Pulvérisateur selon la revendication 3, caractérisé par le fait que la mèche (20) porte contre un bord limite de la plaque de pulvérisation (1) avec une tension mécanique préalable.

5. Pulvérisateur selon une ou plusieurs des revendications antérieures, caractérisé par le fait que le réservoir à liquide (17) qui est de préférence transparent comporte une ouverture (18) servant d'ouverture de remplissage ainsi que d'ouverture d'introduction pour la mèche ou le tube à mèche (19), et qu'avec la mèche il est susceptible d'être enlevé du boîtier et d'y être introduit.

6. Pulvérisateur selon une ou plusieurs revendications antérieures, caractérisé par le fait qu'une tête de pulvérisation (3), de préférence en forme d'entonnoir et susceptible d'être montée sur le boîtier du pulvérisateur, présente pour la plaque de pulvérisation (13) et pour la mèche (20) qui porte contre la plaque de pulvérisation une ouverture (22/23) présentant une arête limite (24) qui applique la mèche contre la plaque de pulvérisation.

**Claims**

1. Liquid-atomizer comprising a vibration generator preferably a vibration generator (9) generating ultrasonic flexural vibrations, which generator is coupled with a diffuser plate (13), further comprising a container for liquids (17) and a flexible element (20) interposed between said container and said diffuser plate for feeding of the liquid by capillary action, characterized in that said element is a wick (20) of a flexible yielding material adjoining with its free end with a mechanical tension against said diffuser plate (13).

2. Liquid-atomizer conforming claim 1 further characterized in that said wick (20) is installed and leaded next to its coupling point in a wick-tube (19) of an essentially rigid material.

3. Liquid-atomizer conforming claim 1 or 2 further characterized in that said wick (20) adjoins punctiformly or in line at said diffuser plate (13).

4. Liquid-atomizer conforming claim 3, further characterized in that said wick (20) adjoins at an edge of said diffuser plate (13) with a mechanical tension.

5. Liquid-atomizer conforming to one or some claims mentioned above further characterized in that said preferably transparent container for liquids (17) has an opening (18) for filling of the liquid and lead-in of the wick (20) resp. of the tube (19) of said wick and in that said container together with the wick is adapted for removing of the housing of the atomizer and for leading in this housing.

6. Liquid-atomizer conforming to one or some claims mentioned above further characterized in that a preferably conical spraying head (3) for the diffuser plate (13) and for the wick (20) adjoining at the diffuser plate and adapted for mounting on the housing (2) of the atomizer has a recess (22/23) with an edge (24), pressing said wick against the diffuser plate.